# EUROPEAN PATENT SPECIFICATION

(11) **EP 1 518 574 B1**
(45) Date of publication and mention of the grant of the patent: **26.08.2009**
(21) Application number: 04021121.1
(22) Date of filing: 06.09.2004
(51) Int. Cl.: A61M 31/00, A61M 3/02

(54) **Cannula for dispensing fluid products for vaginal and anal applications**
Kanüle zur Abgabe von flüssigen Produkten zur analen und vaginalen Anwendung
Canule de distribution de produits fluides pour applications vaginales ou anales

(30) Priority: 26.09.2003 IT MO20030264
(43) Date of publication of application: 30.03.2005
(73) Proprietor: LAMEPLAST S.p.A., 41016 Novi di Modena Rovereto sul Secchia (IT)
(72) Inventor: Fontana, Antonio, 41012 Carpi Prov. of Modena (IT)
(74) Representative: Brogi, Graziano

(56) References cited:
- EP-B- 0 977 612
- WO-A-03/062672
- FR-A- 2 409 761
- FR-A- 2 821 563
- US-A- 2 630 804
- US-A- 4 300 678
- US-A- 4 636 202
- US-A- 5 045 058

## Description

The present invention relates to a cannula for dispensing fluid products for vaginal and anal applications, particularly for the application of ointments, creams, medicinal pastes, or the like.

Cannulas for dispensing fluid medicinal products are known which are used in particular for vaginal and anal applications and are generally sold in packages together with tubes or bottles containing one of said products.

Known cannulas are constituted by a barrel that is suitable to contain the product and inside which a plunger, rigidly coupled to the end of a pusher rod, can slide.

The barrel has an open end, which can be coupled to the dispensing outlet of the tube in order to introduce in the barrel the amount of product to be applied, and through which the introduced product is dispensed.

The opposite end of the barrel is closed by a back wall, which is provided with a hole in which the pusher rod is inserted slidingly; said back wall acts as an element for stopping the plunger in order to prevent its extraction.

The product introduced in the cannula is dispensed by acting on the pusher rod in the direction in which the plunger slides toward the open end of the barrel.

Cannulas are also known which are constituted by a barrel, which has open opposite ends and inside which a plunger is inserted slidingly, and by a pusher rod, which is separate from the plunger and can be coupled detachably thereto.

Undercuts or shoulders for abutment are formed at the opposite ends of the barrel and are suitable to stop the sliding of the plunger, preventing it from being extracted under the action of the pusher rod.

These last cannulas can be sold in packages that contain a single pusher rod, a plurality of empty single-use barrels to be used for the various applications, and one or more tubes of product.

In this case, one of the two ends of the barrels can be coupled to the dispensing outlet of the tube in order to introduce the product in said barrels, while the opposite end acts as a passage for the pusher rod.

As an alternative, said cannulas can be sold in packages that contain a single pusher rod and a plurality of barrels that are already filled with the product to be applied and are provided, at their two opposite ends, with closure plugs that are removed upon use.

However, these known cannulas are not free from drawbacks, including the fact that they have rods that have a significant space occupation, which makes them difficult and expensive to prepare and package and awkward to handle for users.

Another drawback of known cannulas, particularly those constituted by a rod that is detachably associable with the plunger, consists in that they are not straightforward and ready to use for users, since they in fact require additional assembly operations.

Patent documents US 4,636,202 and US 2,630,804 disclose medicament applicators or dispensers which are provided with a plunger assembly having a telescopic structure, thereby when the applicator or dispenser is effectively used the plunger-is handled for assuming from a retracted configuration an elongated configuration suitable for the dispensing of the medicament.

It is also mentioned patent document US 5.045,058 which provides an apparatus, for the cleansing and antisepticising of the vagina, which can be operated as a syringe and which comprises an outer casing enclosing a solid soap material and a telescopic plunger fitted inside a hollow core formed in the solid soap material wherein the telescopic plunger is provided for slidably engaging with the casing for delivering into the vagina a lather obtained by moistening the solid soap material.

The aim of the present invention is to eliminate the drawbacks noted above of known cannulas, by providing a cannula for dispensing fluid products for vaginal and anal applications that is compact and allows to facilitate its preparation and packaging, containing the associated costs.

Within this aim, an object of the present invention is to provide a cannula that is simple and straightforward to use for user.

Another object of the present invention is to provide a cannula that is simple, relatively easy to provide in practice, safe in use, effective in operation, and has a relatively low cost.

This aim and these and other objects that will become better apparent hereinafter are achieved by the present cannula for dispensing fluid products for vaginal and anal applications, comprising a tubular body for containing a fluid product, which is provided, at a first end, with an outlet for dispensing said product and, at a second end that lies opposite the first end, with a back wall in which there is an opening, a plunger, which is inserted so that it can slide axially in said tubular body, and a pusher rod, which is associable with said plunger and can be inserted slidingly within said opening, characterized in that said rod is of the telescopic type and is suitable to assume a retracted configuration, said cannula being in the closed packaging configuration, and at least one elongation configuration, said cannula being in the open configuration for use.

Further characteristics and advantages of the present invention will become better apparent from the following detailed description of a preferred but not exclusive embodiment of a cannula for dispensing fluid products for vaginal and anal applications, illustrated by way of nonlimiting example in the accompanying drawings, wherein:
Figure 1 is a perspective view of the cannula according to the invention, in the closed packaging configuration, with the rod in the retraction configuration;
Figure 2 is a perspective view of the cannula according to the invention, in the open configuration for use, with the rod in the retraction configuration;
Figure 3 is a schematic sectional view of the cannula according to the invention, in the closed packaging configuration, with the rod in the retraction configuration;
Figure 4 is a schematic sectional view of the cannula according to the invention, in the open configuration for use, with the rod in the elongation configuration;

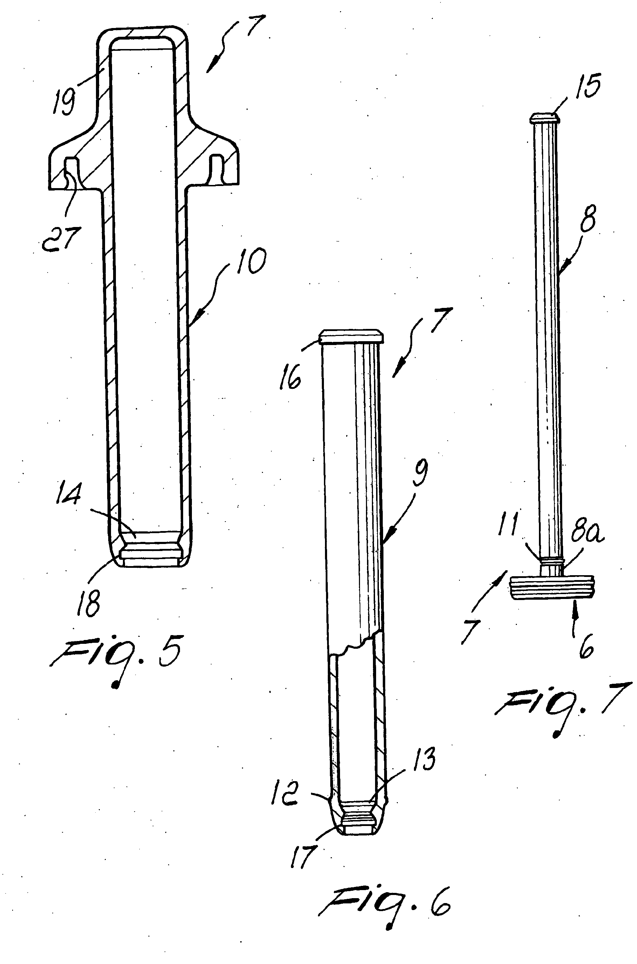

Figure 5 is a schematic sectional view of the outer tubular portion of the rod of the cannula according to the invention;
Figure 6 is a schematic and partially sectional view of the inner tubular portion of the rod of the cannula according to the invention;
Figure 7 is a view of the stem of the rod of the cannula according to the invention.

With reference to the figures, the reference numeral 1 generally designates a cannula for dispensing fluid products P for vaginal and anal applications, particularly for the application of ointments, creams, medicinal pastes, or the like.

The cannula 1 comprises a tubular body 2 for containing the product P, which is provided, at a first end 2a, with an outlet 3 for dispensing the product P and, at a second end 2b that lies opposite the first end, with a back wall 4 in which an opening 5 is formed.

A plunger 6 is inserted in the tubular body 2 so that it can slide axially, and a pusher rod 7 is associated with said plunger and can be inserted slidingly in the opening 5.

The rod 7 is of the telescopic type; in the particular embodiment shown in the figures cited above, it comprises a stem 8, which has an end 8a that is formed monolithically with the plunger 6, an inner tubular body 9, which is fitted on the stem 8 substantially coaxially and so that it can slide axially, and an outer tubular portion 10, which is mounted substantially coaxially and so that it can slide axially on the inner tubular portion 9.

The cannula 1 is sold full of product P in the closed packaging configuration, with the rod 7 in the retraction configuration, in which the stem 8 is retracted into the inner tubular portion 9, which is retracted into the outer tubular portion 10.

When the cannula 1 is in the open configuration for use, the rod 7 assumes an elongation configuration, in which the stem 8 is extended outside the inner tubular portion 9, which is extended outside the outer tubular portion 10.

The cannula 1 comprises means for temporarily retaining the stem 8, the inner tubular portion 9 and the outer tubular portion 10 in the retracted configuration of the rod 7, and means for locking the stem 8, the inner tubular portion 9 and the outer tubular portion 10 in the elongation configuration of the rod 7.

The temporary retention means can be of the interlocking type and can be constituted for example by annular protrusions 11 and 12 formed so as to protrude respectively on the outer lateral surface of the stem 8 and of the inner tubular portion 9 and are coupled to complementary grooves 13 and 14 formed on the inner lateral surface of the inner tubular portion 9 and of the outer tubular portion 10 respectively.

The locking means can be of the interlocking type and can be constituted for example by contoured heads 15 and 16, which are formed at the opposite ends, with respect to the plunger 6, of the stem 8 and of the inner tubular portion 9, respectively, and can be coupled to complementary seats 17 and 18 formed respectively in the inner tubular portion 9 and in the outer tubular portion 10.

A grip is formed at the end of the outer tubular portion 10 that lies opposite with respect to the plunger 6 and is constituted for example by a knob 19 formed monolithically therewith.

The cannula 1 further comprises means for capping the tubular body 2 and the rod 7 in the retraction configuration, which can be constituted for example by a covering sheath 20, which has a closed end 20a and an open opposite end 20b and which, when said cannula is in the closed packaging configuration, is fitted detachably over the tubular body 2 and on at least one portion of the rod 7 in the retraction configuration.

The dispensing outlet 3 is associated with detachable closure means, which can be constituted for example by a plug 21, which is formed inside the jacket 20 at its closed end 20a.

Finally, the cannula 1 comprises detachable tamper-resistant means 22, which seal it in the closed packaging configuration.

The tamper-resistant means 22 can comprise for example a tab 23, which can be removed by tearing, is wrapped around the outer tubular portion 10, and is associated, along respective tear lines 24 and 25 formed along its opposite longitudinal edges, with the sheath 20 and with a strip 26 that is rigidly coupled to the knob 19.

The strip 26 comprises a fixing flap 26a, which is rigidly coupled to the knob 19, for example by interlocking in a seat 27 that is formed along the perimetric edge of said knob, and an indicator flap 26b, which protrudes from the seat 27 to indicate that the cannula 1 has been opened for the first time.

As an alternative, the fixing flap 26a might be rigidly coupled to the knob 19 by way of systems other than interlocking, for example by gluing, welding, ultrasonic welding, or others.

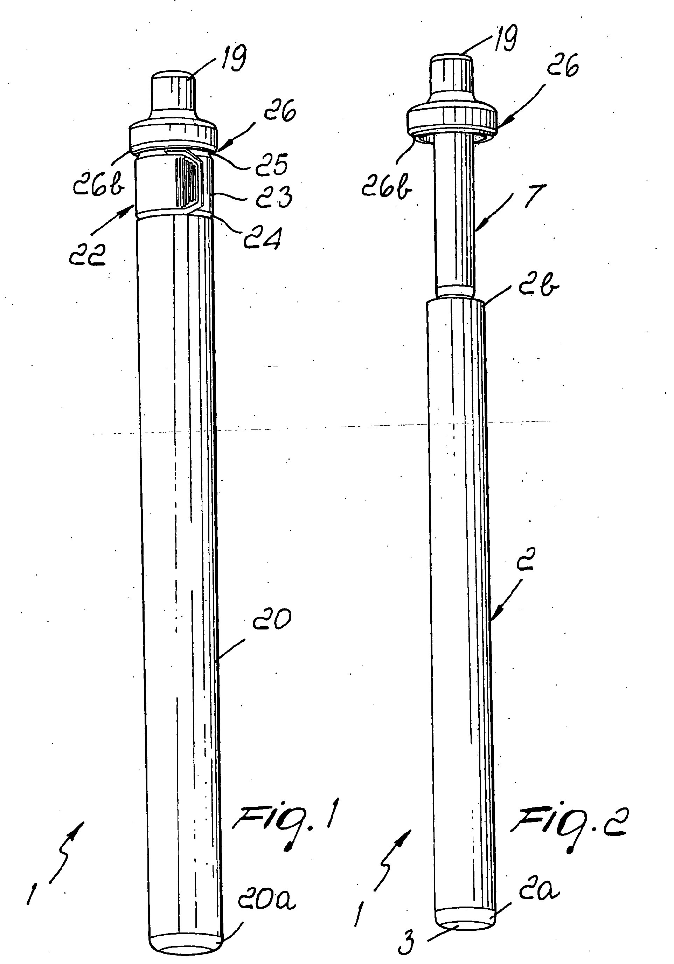

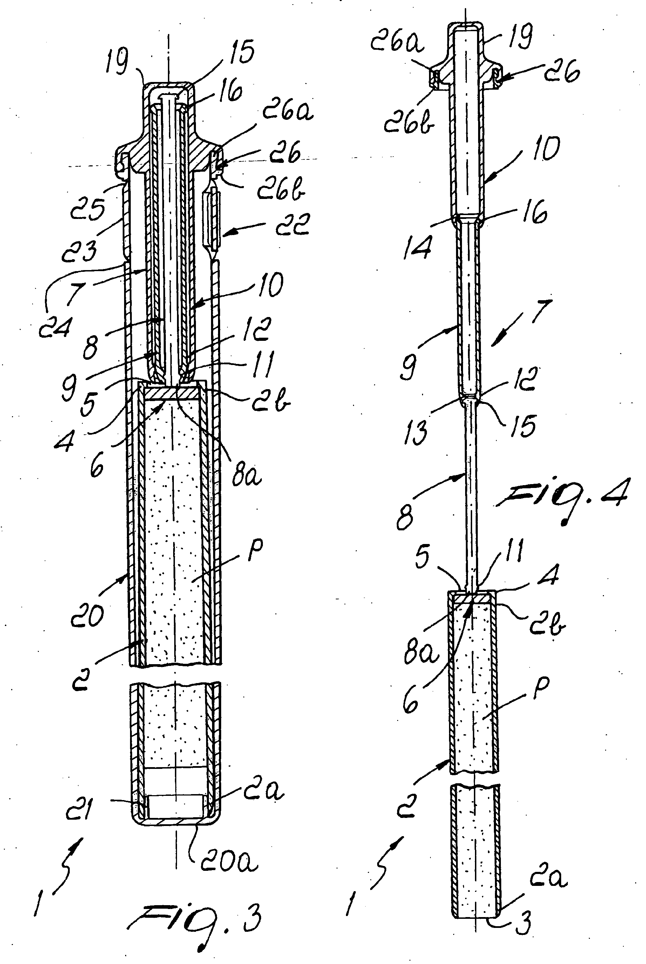

The cannula 1 is sold in the closed packaging configuration (Figures 1 and 3), filled with product P; in this configuration, the rod 7 is locked temporarily in the retraction configuration by the annular protrusions 11 and 12 coupled to the corresponding grooves 13 and 14, while the sheath 20 caps the tubular body 2 and a portion of said rod and closes the dispensing outlet 3 with the plug 21.

The sheath 20 is anchored temporarily to the knob 19 by way of tamper-resistant removable means 22.

By removing the tab 23, the sheath 20 is disengaged from the knob 19, while the strip 26 remains coupled to the knob 19, with the indicator flap 26b that protrudes from it so as to indicate that first opening has occurred.

By sliding off the sheath 20, the tubular body 2 is exposed and the dispensing outlet 3 is connected to the outside, arranging the cannula 1 in the open configuration for use, ready for application of the product P (Figures 2 and 4).

In this configuration, it is sufficient to grip the knob 19 and pull in order to extract, in succession one after the other, the inner tubular portion 9 from the outer tubular portion 10 and the stem 8 from the inner tubular portion 9, moving the rod 7 into the elongation configuration.

The interlocking of the heads 15 and 16 in the corresponding seats 17 and 18 locks the rod 7 in the elongation configuration; the rod 7 thus assumes a rigid configuration, which is capable of transmitting to the plunger 6 the thrust action that the user applies to it by means of the knob 19 in the direction for sliding toward the dispensing outlet 3 for the application of the product P.

In practice, it has been found that the described invention achieves the intended aim and objects.

The cannula according to the invention is in fact compact, particularly with respect to known cannulas provided with a pusher rod formed by means of a single rigid body, and this compactness makes it easier to prepare, package and handle.

The invention thus conceived is susceptible of numerous modifications and variations, all of which are within the scope of the appended claims.

All the details may further be replaced with other technically equivalent ones.

In practice, the materials used, as well as the shapes and the dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A cannula (1) for dispensing fluid products (P) for vaginal and anal applications, comprising :
a tubular body (2) for containing a fluid product, which is provided, at a first end (2a), with an outlet (3) for dispensing said product and, at a second end (2b) that lies opposite the first end (2a), with a back wall (4) in which there is an opening (5),
a plunger (6), which is inserted so that it can slide axially in said tubular body (2), and
a pusher rod (7,8, 9, 10, 19), which is associated with said plunger (6) and is inserted slidingly within said opening (5),
wherein said pusher rod (7, 8, 9, 10) is of the telescopic type and is adapted to assume a retracted configuration, with said cannula (1) being in the closed packaging configuration, and at least one elongated configuration, with said cannula (1) being in the open configuration for use,
said cannula (1) being **characterise in that** it comprises detachable tamper-resistant means (22, 23, 24, 26) for sealing said cannula (1) in said closed packaging configuration,
wherein said detachable tamper-resistant means (22, 23) are rigidly coupled to a grip (19) which is formed at the end of an outer tubular portion (10) of said pusher rod (7) that lies opposite said plunger (6).

2. The cannula (1) according to claim 1, **characterized in that** said pusher rod (7) comprises a stem (8), which is provided with an end (8a) that is formed monolithically with said plunger (6), and **in that** said outer tubular portion (10) is fitted on said stem (8) substantially coaxially so that it can slide axially.

3. The cannula according to claim 2, **characterized in that** said pusher rod (7) comprises at least one inner tubular portion (9), which is interposed between said outer tubular portion (10) and said stem (8) and is associated therewith substantially coaxially so that it can slide axially.

4. The cannula according to claims 2 or 3, **characterized in that** it comprises means (11, 12, 13, 14) for temporarily retaining said stem (8) and said tubular portion or portions (9, 10) of the rod (7) in said retracted configuration.

5. The cannula according to claim 4, **characterized in that** said temporary retention means (11, 12, 13, 14) are of the interlocking type.

6. The cannula according to claim 5 as dependent from claim 2, **characterized in that** said temporary retention means comprise at least one protrusion, which is formed so as to protrude on the outer lateral surface of said stem (8) and is coupled to a complementary groove formed on the inner lateral surface of said outer tubular portion (10).

7. The cannula according to claim 5 as dependent from claim 3, **characterized in that** said temporary retention means comprise at least one protrusion (11), which is formed so as to protrude on the outer lateral surface of said stem (8) and is coupled to a complementary groove (13) formed on the inner lateral surface of said inner tubular portion (9), and at least one protrusion (12), which is formed so as to protrude on the outer lateral surface of said inner tubular portion (9) and is coupled to a complementary groove (14) formed on the inner lateral surface of said outer tubular portion (10).

8. The cannula according to claims 2 or 3, **characterized in that** it comprises means (15, 16, 17, 18) for locking said stem (8) and said tubular portions (9, 10) of the rod (7) in said elongated configuration.

9. The cannula according to claim 8, **characterized in that** said locking means (15, 16, 17, 18) are of the interlocking type.

10. The cannula according to claim 9 as dependent from claim 2, **characterized in that** said locking means comprise a contoured head, which is formed at the end of said stem (8) that lies opposite said plunger (6) and can be coupled to a complementary seat formed in said outer tubular portion (10).

11. The cannula according to claim 9 as dependent from claim 3, **characterized in that** said locking means comprise a contoured head (15), which is formed at the end of said stem (8) that lies opposite said plunger (6) and can be coupled to a complementary seat (17) formed in said inner tubular portion (19), and a countering head (16) which is formed at the end of said inner tubular portion (9) that lies opposite said plunger (6) and can be coupled to a complementary seat (18) formed in said outer tubular portion (10).

12. The cannula according to claims 2 or 3, **characterized in that** said grip comprises a knob (19) that is formed monolithically at said end of the outer tubular portion that lies opposite said plunger (6).

13. The cannula according to one or more of the preceding, **characterized in that** it comprises means (20) for capping said tubular body (2) and said pusher rod (7) in the retracted configuration.

14. The cannula according to claim 13, **characterized in that** said capping means comprise a sheath (20), which has a closed end (20a) and an opposite open end (20b) and is fitted detachably over said tubular body (2) and over at least one portion of said pusher rod (7) in the retracted configuration.

15. The cannula according to claim 14, **characterized in that** said detachable tamper-resistant means (22) comprise a tab (23) that can be removed by tearing and is wound around said outer tubular portion (10) and is associated, along respective tear lines (24, 25) formed along its opposite longitudinal edges, with said sheath (20) and with a strip (26) that is rigidly coupled to said grip (19).

16. The cannula according to claim 15, **characterized in that** said strip (26) comprises a fixing flap (26a), which is rigidly associated with the perimetric edge of said grip (19), and an indicator flap (26b), which protrudes from said edge.

17. The cannula (1) according to claim 13, **characterized in that** it comprises detachable means (21), associated with said capping means (20), for closing said dispensing outlet (3).

18. The cannula according to claim 17, **characterized in that** said detachable closure means comprise a plug (21), which is formed inside a sheath (20) of said capping means at a closed end thereof (24a).

19. The cannula according to one or more of the preceding claims, wherein said pusher rod (7) comprises a stem (8), which is provided with an end (8a) that is associated with said plunger (6), **characterized in that** said end (8a) of the stem (8) associated with said plunger (6) is formed monolithically therewith.

## Patentansprüche

1. Kanüle (1) zur Abgabe von flüssigen Produkten (P) für vaginale und anale Anwendungen mit:
einem Röhrenkörper (2) zum Aufbewahren eines flüssigen Produktes, der - an einem ersten Ende (2a) - mit einem Auslass (3) zur Abgabe des Produktes bereitgestellt ist, und - an einem zweiten Ende (2b), welches dem ersten Ende (2a) gegenüber liegt - mit einer Rückwand (4) versehen ist, in der sich eine Öffnung (5) befindet,
einem Plungerkolben (6), der so eingesetzt ist, dass er sich in den Röhrenkörper (2) axial verschieben kann, und mit
einem Stößelstab (7, 8, 9, 10, 19), der mit dem Plungerkolben (6) in Verbindung steht und in die genannte Öffnung (5) verschiebbar eingesetzt wird,
**gekennzeichnet dadurch, dass** der genannte Stößelstab (7, 8, 9, 10) teleskopisch ausziehbar und so ausgelegt ist, um eine eingezogene Betriebskonfiguration anzunehmen, wobei sich die Kanüle (1) in der geschlossenen Packanordnung befindet, und **gekennzeichnet durch** mindestens eine Verlängerungsanordnung, wobei sich die Kanüle (1) in der geöffneten Betriebskonfiguration für eine Anwendung befindet,
und die Kanüle (1) **dadurch gekennzeichnet ist**, dass sie abnehmbare, manipulationssichere Mittel (22, 23, 24, 26) zum versiegeln der Kanüle (1) in der geschlossenen Packanordnung aufweist,
worin die abnehmbaren, manipulationssicheren Mittel (22, 23) mit einem Griff (19) starr verbunden sind, der am Ende eines äußeren Röhrenabschnitts (10) des Stößelstab (7) ausgebildet ist, das zum Plungerkolben (6) gegenüberliegend angeordnet ist.

2. Kanüle (1) gemäß Anspruch 1, **dadurch gekennzeichnet, dass** der Stößelstab (7) einen Schaft (8) aufweist, der mit einem Ende (8a) versehen ist, das mit dem Plungerkolben (6) monolithisch ausgebildet ist, und **dadurch gekennzeichnet, dass** der äußere Röhrenabschnitt (10) über dem Schaft (8) im Wesentlichen so koaxial eingepasst ist, dass er axial gleiten kann.

3. Kanüle gemäß Anspruch 2, **dadurch gekennzeichnet, dass** der Stößelstab (7) mindestens einen röhrenförmigen Innenabschnitt (9) aufweist, der sich zwischen dem äußeren Röhrenabschnitt (10) und dem Schaft (8) befindet und mit diesem im Wesentlichen so koaxial verbunden ist, dass er axial gleiten kann.

4. Kanüle gemäß den Ansprüchen 2 oder 3**, dadurch gekennzeichnet, dass** sie Mittel (11, 12, 13, 14) zum temporären Befestigen des Schaftes (8) und der Röhrenabschnitte (9, 10) des Stößelstabs (7) in der eingezogenen Betriebskonfiguration aufweist.

5. Kanüle gemäß Anspruch 4, **dadurch gekennzeichnet, dass** die temporären Befestigungsmittel (11, 12, 13, 14) einen ineinandergreifenden Sperrmechanismus aufweisen.

6. Kanüle gemäß Anspruch 5 in Abhängigkeit von Anspruch 2, **dadurch gekennzeichnet, dass** die temporären Befestigungsmittel mindestens einen vorsprung aufweisen, der so ausgebildet ist, dass er an der seitlichen Außenfläche des Schaftes (8) hervorsteht und mit einer komplementären Kerbe gekoppelt wird, die an der seitlichen Innenfläche des äußeren Röhrenabschnitts (10) ausgestaltet ist.

7. Kanüle gemäß Anspruch 5 in Abhängigkeit von Anspruch 3, **dadurch gekennzeichnet, dass** die temporären Befestigungsmittel mindestens einen Vorsprung (11) aufweisen, der so ausgebildet ist, dass er an der seitlichen Außenfläche des Schaftes (8) hervorsteht und mit einer komplementären Kerbe (13) gekoppelt wird, die an der seitlichen Innenfläche des inneren Röhrenabschnitts (9) ausgestaltet ist, und mit mindestens einem Vorsprung (12) versehen ist, der so ausgestaltet ist, dass er an der seitlichen Außenfläche des inneren Röhrenabschnitts (9) hervorsteht und mit einer komplementären Kerbe (14) gekoppelt wird, die an der seitlichen Innenfläche des äußeren Röhrenabschnitts (10) ausgebildet ist.

8. Kanüle gemäß den Ansprüchen 2 oder 3, **dadurch gekennzeichnet, dass** sie Mittel (15, 16, 17, 18) zum Fixieren des Schaftes (8) und der Röhrenabschnitte (9, 10) des Stößelstab (7) in der verlängerten Betriebskonfiguration aufweist.

9. Kanüle gemäß Anspruch 8**, dadurch gekennzeichnet, dass** die Fixiermittel (15, 16, 17, 18) einen ineinandergreifenden Sperrmechanismus aufweisen.

10. Kanüle gemäß Anspruch 9 in Abhängigkeit von Anspruch 2, **dadurch gekennzeichnet, dass** die Fixiermittel ein passend profiliertes Kopfteil aufweisen, das am Ende des Schaftes (8) ausgebildet ist, das zum Plungerkolben (6) gegenüberliegend angeordnet ist und das mit einem komplementären Einpasssitz gekoppelt werden kann, der in dem äußeren Röhrenabschnitt (10) ausgebildet ist.

11. Kanüle gemäß Anspruch 9 in Abhängigkeit von Anspruch 3,
**dadurch gekennzeichnet, dass** die Fixiermittel ein passend profiliertes Kopfteil (15) aufweisen, das am Ende des Schaftes (8) ausgebildet ist, das zum Plungerkolben (6) gegenüberliegend angeordnet ist und mit einem komplementären Einpasssitz (17) gekoppelt werden kann, der in dem inneren Röhrenabschnitt (19) ausgestaltet ist, und ein konturierte Kopfteil (16), das am Ende des inneren Röhrenabschnitts (9) ausgebildet ist, das zum Plungerkolben (6) gegenüberliegend angeordnet ist und mit einem komplementären Einpasssitz (18) gekoppelt werden kann, der in dem äußeren Röhrenabschnitt (10) ausgebildet ist.

12. Kanüle gemäß den Ansprüchen 2 oder 3, **dadurch gekennzeichnet, dass** der Griff einen Knauf (19) aufweist, der am Ende des äußeren Röhrenabschnitts monolithisch ausgebildet ist, welcher zum Plungerkolben (6) gegenüberliegend angeordnet ist.

13. Kanüle gemäß einem oder mehreren der vorhergehenden Ansprüche, **dadurch gekennzeichnet, dass** sie Mittel (20) zum Verkappen des Röhrenkörpers (2) und des Stößelstab (7) in der eingezogenen Betriebskonfiguration aufweist.

14. Kanüle gemäß Anspruch 13, **dadurch gekennzeichnet, dass** die Verkappungsmittel eine Ummantelung (20) aufweisen, die ein geschlossenes Ende (20a) und ein entgegengesetzt offenes Ende (20b) umfasst, und die in der eingezogenen Betriebskonfiguration über dem Röhrenkörper (2) und über mindestens einem Abschnitt des Stößelstabs (7) entfernbar eingepasst ist.

15. Kanüle gemäß Anspruch 14, **dadurch gekennzeichnet, dass** die abnehmbaren, manipulationssicheren Mittel (22) eine Lasche (23) aufweisen, die durch Abriss entfernt werden kann, die um den äußeren Röhrenabschnitt (10) gewunden ist und die längs der jeweiligen Reißlinien (24, 25), welche entlang den sich gegenüberliegenden Längsrändern des äußeren Röhrenabschnitts ausgebildet sind, mit der Ummantelung (20) und mit einem Bundring (26) verbunden ist, wobei dieser mit dem Griff (19) starr verbunden ist.

16. Kanüle gemäß Anspruch 15, **dadurch gekennzeichnet, dass** der Bundring (26) eine Befestigungsklappe (26a) aufweist, die mit dem Perimeterrand des Griffes (19) starr verbunden ist, und eine Indikatorklappe (26b) umfasst, die von dem Rand hervorsteht.

17. Kanüle (1) gemäß Anspruch 13, **dadurch gekennzeichnet, dass** sie abnehmbare Mittel (21) aufweist, die mit den Verkappungsmitteln (20) in Verbindung stehen, um die Spenderausgabe (3) zu verschließen.

18. Kanüle gemäß Anspruch 17, **dadurch gekennzeichnet, dass** die abnehmbaren Verschlussmittel einen Abdichtstopfen (21) umfassen, welcher im Innern der Ummantelung (20) der Verkappungsmittel an deren geschlossenem Ende (20a) ausgestaltet ist.

19. Kanüle gemäß einem oder mehreren der vorhergehenden Ansprüche, **gekennzeichnet dadurch, dass** der Stößelstab (7) einen Schaft (8) aufweist, der mit einem Ende (8a) versehen ist, das mit dem Plungerkolben (6) verbunden ist, **dadurch gekennzeichnet, dass** das Ende (8a) des Schaftes (8), das mit dem Plungerkolben (6) verbunden ist, mit diesem monolithisch ausgebildet ist.

## Revendications

1. Canule (1) pour la distribution de produits fluides (P) pour applications vaginales et anales, comprenant:
un corps tubulaire (2) pour contenir un produit fluide qui est pourvu, à une première extrémité (2a), d'une bouche (3) de distribution dudit produit et, à une deuxième extrémité (2b), opposée à la première (2a), d'un fond (4) dans lequel est définie une ouverture (5),
un piston (6), inséré de manière axialement coulissant dans ledit corps tubulaire (2), et
une tige de poussée (7, 8, 9, 10, 19), associable audit piston (6) et insérable de manière coulissante dans ladite ouverture (5), où ladite tige de poussée (7, 8, 9, 10) est du type télescopique et apte à assumer une position retirée, avec ladite canule (1) en étant dans la configuration fermée d'emballage, et au moins une configuration d'élongation, avec ladite canule (1) en étant dans la configuration ouverte d'emploi,
ladite canule ***caractérisée en ce qu*'**elle comprend des moyens détachables de fermeture inviolable (22, 23, 24, 26) pour sceller ladite canule (1) dans ladite configuration fermée d'emballage, avec lesdits moyens détachables de fermeture inviolable (22, 23) qui sont rigidement accouplés avec une poignée (19), formée à l'extrémité d'une portion tubulaire extérieure (10) de ladite tige de poussée (7), qui se trouve opposée au susdit piston (6).

2. Canule (1) selon la revendication 1, ***caractérisée en ce que*** ladite tige de poussée (7) comprend une tige (8), pourvue d'une extrémité (8a) formée de manière monolithique avec ledit piston (6), et dans ladite portion tubulaire extérieure (10) est insérée ladite tige (8) de manière sensiblement coaxiale pour qu'elle puisse coulisser axialement.

3. Canule selon la revendication 2, ***caractérisée en ce que*** ladite tige de poussée (7) comprend au moins une portion tubulaire intérieure (9), interposée entre ladite portion tubulaire extérieure (10) et ladite tige (8) et associée à elles de manière sensiblement coaxiale et coulissant axialement.

4. Canule selon la revendication 2 ou 3, ***caractérisée en ce qu*'**elle comprend des moyens (11, 12, 13, 14) de retenue temporaire de ladite tige (8) et de ladite portion tubulaire ou portions (9, 10) de la tige de poussée (7) dans ladite configuration retirée.

5. Canule selon la revendication 4, ***caractérisée en ce que*** lesdits moyens de retenue temporaire (11, 12, 13, 14) sont du type à emboitement.

6. Canule selon la revendication 4 et dépendant de la revendication 2, ***caractérisée en ce que*** lesdits moyens de retenue temporaire comprennent au moins une saillie, formée pour saillir de la surface extérieure de ladite tige (8) et accouplée avec une gorge complémentaire formée sur la surface latérale intérieure de ladite portion tubulaire extérieure (10).

7. Canule selon la revendication 5 et dépendant de la revendication 3, ***caractérisée en ce que*** lesdits moyens de retenue temporaire comprennent au moins une saillie (11), formée pour saillir de la surface extérieure de ladite tige (8) et accouplée avec une gorge complémentaire (13) formée sur la surface latérale intérieure de ladite portion tubulaire intérieure (9), et au moins une saillie (12), formée pour saillir de la surface latérale extérieure de ladite portion tubulaire intérieure (9) et accouplée avec une gorge complémentaire (14) formée sur la surface latérale intérieure de ladite portion tubulaire extérieure (10).

8. Canule selon les revendications 2 ou 3, ***caractérisée en ce qu*'**elle comprend des moyens de blocage (15, 16, 17, 18) pour bloquer ladite tige (8) et lesdites portions tubulaires (9, 10) de ladite tige de poussée (7) dans ladite configuration d'élongation.

9. Canule selon la revendication 8, ***caractérisée en ce que*** lesdits moyens de blocage (15, 16, 17, 18) sont du type à emboitement.

10. Canule selon la revendication 9 et dépendant de la revendication 2, ***caractérisée en ce que*** lesdits moyens de blocage comprennent une tête façonnée, formée à l'extrémité de ladite tige (8) opposée audit piston (6) et qui peut être accouplée avec un logement complémentaire formé dans ladite portion tubulaire extérieure (10).

11. Canule selon la revendication 9 et dépendant de la revendication 3, ***caractérisée en ce que*** lesdits moyens de blocage comprennent une tête façonnée (15), formée à l'extrémité de ladite tige (8) opposée audit piston (6) et qui peut être accouplée avec un logement complémentaire (17) formé dans ladite portion tubulaire intérieure (19), et une tête façonnée (16), formée à l'extrémité de ladite portion tubulaire intérieure (9) opposée audit piston (6) et qui peut être accouplée avec un logement complémentaire (18) formé dans ladite portion tubulaire extérieure (10).

12. Canule selon les revendications 2 ou 3, ***caractérisée en ce que*** ladite poignée comprend une poignée (19) formée de manière monolithique avec l'extrémité de ladite portion tubulaire extérieure opposée au piston (6).

13. Canule selon l'une ou plusieurs des revendications précédentes, ***caractérisée en ce qu'***elle comprend des moyens (20) pour encapuchonner ledit corps tubulaire (2) et ladite tige de poussée (7) dans la configuration retirée.

14. Canule selon la revendication 13, ***caractérisée en ce que*** lesdits moyens pour encapuchonner comprennent une gaine (20), qui a une extrémité fermée (20a) et l'extrémité opposée ouverte (20b) qui est placée de manière détachable sur ledit corps tubulaire (2) et sur au moins une portion de ladite tige de poussée (7) dans la configuration retirée.

15. Canule selon la revendication 14, ***caractérisée en ce que*** lesdits moyens détachables de fermeture inviolable (22) comprennent une bandelette (23) qu'on peut enlever en la déchirant et qui est enroulé autour de ladite portion tubulaire extérieure (10) et qui est associée, le long des lignes de déchirure (24, 25) formées le long de ses bords longitudinaux opposés, à ladite gaine (20) et à une bande (26) solidaire avec ladite poignée (19).

16. Canule selon la revendication 15, ***caractérisée en ce que*** ladite bande (26) comprend un ourlet (26a), fixé solidairement au bord périmétrique de ladite poignée (19), et un ourlet indicateur (26b) saillant dudit bord.

17. Canule (1) selon la revendication 13, ***caractérisée en ce qu*'**elle comprend des moyens détachables (21), associées à ladite gaine (20), pour fermer ladite bouche de distribution (3).

18. Canule selon la revendication 17, ***caractérisée en ce que*** lesdits moyens détachables de fermeture comprennent un bouchon formé à l'intérieur d'une gaine (20) desdits moyens pour encapuchonner en correspondance avec son extrémité fermée (20a).

19. Canule selon l'une ou plusieurs des revendications précédentes avec ladite tige de poussée (7) qui comprend une tige (8) pourvue d'une extrémité (8a) associée audit piston (6) ***caractérisée en ce que*** ladite extrémité (8a) de la tige (8) associée audit piston (6) est formée de manière monolithique.
